(19) Europäisches Patentamt
European Patent Office
Office européen des brevets

(11) Veröffentlichungsnummer: **0 113 467 B1**

(12) # EUROPÄISCHE PATENTSCHRIFT

(45) Veröffentlichungstag der Patentschrift: **30.04.86**

(51) Int. Cl.⁴: **C 07 C 47/14**, C 07 C 45/40

(21) Anmeldenummer: **83112735.2**

(22) Anmeldetag: **21.08.81**

(60) Veröffentlichungsnummer der früheren Anmeldung nach Art. 76 EPÜ: **0046735**

(54) **2,2-Dichlor-3,3,3-trifluorpropionaldehyd und Verfahren zu seiner Herstellung.**

(30) Priorität: **27.08.80 CH 6447/80**
**11.06.81 CH 3834/81**

(43) Veröffentlichungstag der Anmeldung: **18.07.84 Patentblatt 84/29**

(45) Bekanntmachung des Hinweises auf die Patenterteilung: **30.04.86 Patentblatt 86/18**

(84) Benannte Vertragsstaaten: **AT BE CH DE FR GB IT LI NL**

(56) Entgegenhaltungen: **Keine**

(73) Patentinhaber: **CIBA-GEIGY AG, Klybeckstrasse 141, CH-4002 Basel (CH)**

(72) Erfinder: **Steiner, Eginhard, Dr., Obere Hofackerstrasse 3, CH-4414 Füllinsdorf (CH)**
Erfinder: **Martin, Pierre, Dr., Meisenweg 38, CH-4310 Rheinfelden (CH)**

EP 0 113 467 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft den 2,2-Dichlor-3,3,3-trifluorpropionaldehyd der Formel III
$CF_3$-$Cl_2$-CHO (III)
und ein Verfahren zu seiner Herstellung.

Der 2,2-Dichlor-3,3,3-trifluorpropionaldehyd ist ein wichtiges Zwischenprodukt bei der Herstellung von 2,3-Dichlor-5-trifluor-pyridin.

Durch Methyl-, Trichlormethyl- oder Trifluormethylgruppen substituierte Chlorpyridine konnten bisher nur durch aufwendige mehrstufige Verfahren hergestellt werden.

2,3-Dichlor-5-methylpyridin kann z. B. dadurch erhalten werden, dass man 2-Chlor-3-amino-5-methylpyridin diazotiert und unter Diazospaltung mit Chlor substituiert. Das genannte Aminopyridin kann durch Chlorierung von 3-Methylpyridin zum 2-Chlor-5-methylpyridin, Nitrierung zum 2-Chlor-3-nitro-methylpyridin und Reduktion der Nitroverbindungen hergestellt werden. Durch Chlorierung des 2 3-Dichlor-5-methylpyridins erhält man das 2,3-Dichlor-5-trichlormethylpyridin, das durch Austausch der Chloratome der Trichlormethylgruppe gegen Fluoratome in das 2,3-Dichlor-5-trifluormethylpyridin überführt werden kann [vgl. z. B. die europäische Patentveröffentlichung 004414].

Chlorpyridine der Formel I

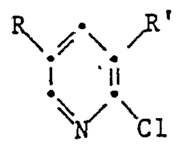

(I),

worin entweder R Chlor und R' methyl oder Trifluormethyl, R Methyl, Trichlormethyl oder Trifluormethyl und R' Chlor oder R und R' Methyl bedeuten, können in der Weise hergestellt werden, dass man in Gegenwart eines Katalysators

a) Trichloracetaldehyd an Methylacrylonitril oder α-Trifluormethylacrylonitril,

b) 2,2-Dichlorpropionaldehyd, Pentachlorpropionaldehyd oder 2,2-Dichlor-3,3,3-trifluorpropionaldehyd an Acrylonitril oder

c) 2,2-Dichlorpropionaldehyd an Methacrylonitril

anlagert und das gebildete Zwischenprodukt der Formel II

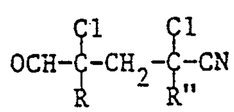

(II),

worin entweder R Chlor und R" Methyl oder Trifluormethyl; R Methyl, Trichlormethyl oder Trifluormethyl und R" Wasserstoff oder R und R" Methyl bedeuten, zu einer Verbindung der Formel I cyclisiert.

2,2-Dichlor-3,3,3-trifluorpropionaldehyd ist nach der vorliegenden Erfindung zugänglich, indem man entsprechende Olefine mit Ozon umsetzt und die Reaktionsmasse reduktiv aufarbeitet. Als Olefin wird der 4,4-Dichlor-5,5,5-trifluor-2-methyl-2-pentencarbonsäuremethylester bevorzugt eingesetzt.

Als Lösungsmittel können verwendet werden: Organische Säuren wie Ameisensäure, Essigsäure, Propionsäure; die Ester dieser Säuren wie Essigsäureäthylester, Essigsäuremethylester, Ameisensäureäthylester Ameisensäuremethylester; aliphatische Kohlenwasserstoffe wie Pentan, Hexan, Heptan, Octan, Cyclopentan, Cyclohexan; chlorierte Kohlenwasserstoffe wie Methylemchlorid, Chloroform, Tetrachlorkohlenstoff; oder auch Wasser.

Die Reaktionstemperaturen betragen je nach der Natur des Lösungsmittels −90°C bis +70°C vorzugsweise jedoch −70°C bis +30°C.

Die reduktive Aufarbeitung der Ozonolyseprodukte kann entweder durch direkte katalytische Hydrierung mit Wasserstoff und einem Edelmetallkatalysator wie Platin, palladium, Rhodium, gegebenenfalls aufgezogen auf ein Trägermaterial, erfolgen oder durch Zusatz von Reduktionsmitteln wie Zink oder Dimethylsulfid.

Eine bevorzugte Ausführungsform dieses Ozonolyseverfahrens besteht darin, dass man 4,4-Dichlor-5,5,5-trifluor-2-methyl-2-pentencarbonsäuremethylester in Essigsäure bei 20°C ozonisiert, dann dem Reaktionsgemisch eine wässrige Aufschlämmung von Zinkpulver zusetzt und den 2,2-Dichlor-3,3,3-trifluorpropionaldehyd direkt aus dem Gemisch abdestilliert.

Die Chlorpyridine der Formel I können auf an sich bekannte Weise über eine oder mehrere Zwischenstufen zur Herstellung verschiedener Wirkstoffe, besonders von Insektiziden und Herbiziden, verwendet werden (vgl. z. B. schweizerisches Patent 622 170, europäische Patentveröffentlichungen Nr. 00176, 00483 und 04414; europäische Patentanmeldung 81810181.8; DE-OS 28 12 649 und 2 748 636; Südafr. Patent 7 802 440; japanische Patentpublikationen 5 4115-380, 5 5038-356, 5 5079-369 und 56-39069 und belgische Patentschrift 862 325).

Das erfindungsgemässe Verfahren wird durch die folgenden Beispiele näher erläutert.

**Beispiel 1:**

**Herstellung von 2,2-Dichlor-3,3,3-trifluorpropionaldehyd**

In eine Lösung von 100,4 g 4,4-Dichlor-5,5,5-trifluor-2-methyl-2-pentencarbonsäuremethylester in 800 ml Eisessig werden 19,2 g Ozon (in Gemisch mit Sauerstoff) bei 20° eingeleitet. Danach setzt man eine Aufschlämmung von 15 g Zinkstaub in 15 ml Wasser zu und destilliert den entstandenen 2,2-Dichlor-3,3,3-trifluorpropionaldehyd bei Normaldruck ab. Man erhält 52,8 g Produkt als farblose, stechend riechende Flüssigkeit, Sdp. 66-67°.

IR ($CCl_4$):$\nu_{CO}$ 1770 $cm^{-1}$

$^1$H-NMR ($CDCl_3$): δ = 9,3 (q, J = 2Hz) ppm.

Analyse: $C_3HCl_2F_30$ (180,9)

berechnet: C 19,92% H 0,56% F 31,50% Cl 39,19%

gefunden: C 20,2% H 0,8% F 30,9% Cl 38,5%.

**Beispiel 2:**

**Herstellung von 4-Formyl-2,4-dichlor-5,5,5-trifluorvaleronitril**

Ein Gemisch von 36 g 2,2-Dichlor-3,3,3-trifluorpropionaldehyd, 80 ml Acetonitril, 80 ml Acrylonitril und 0.5 g Kupfer(I)chlorid wird für 12 Stunden in einem Tantal-Autoklaven auf 120° erhitzt. Nach Aufarbeitung gemäss Beispiel 1a) erhält man das 4-Formyl-2,4-dichlor-5,5,5-trifluorvaleronitril als farbloses Oel, Sdp: 85-86°/900 pa.

IR ($CCl_4$): $\nu_{CN}$ 2550 $cm^{-1}$, $\nu_{CO}$ 1750 $cm^{-1}$.

$^1$H-NMR ($CDCl_3$): δ = 9,56 (m, IH, CHO); 4,7 (m, 1H, C-2-3); 2,7-3,3 (m, 2H, C-3-H) ppm. (Diastereomerengemisch).

Analyse: $C_6H_4Cl_2F_3NO$ (234,0)

berechnet: C 30,80% H 1,73% N 5,99% F 24,36%

gefunden: C 31,5% H 2,0% N 5,9% F 23,8%.

**Beispiel 3**

**Herstellung von 2,3-Dichlor-5-trifluormethylpyridin**

25,0 g des gemäss Beispiel 2 erhaltenen 4-Formyl-2,4-dichlor-5,5,5-trifluorvaleronitrils werden in einem Tantal-Autoklaven mit 0,1 g Kupferpulver für 5 Stunden auf 170° erhitzt.

Durch Wasserdampfdestillation erhält man 11,9 g 2,3-Dichlor-5-trifluor-methylpyridin als farbloses, nach Pfefferminz riechendes Oel, Sdp. 80°/3325 pa.

$^1$H-NMR ($CDCl_3$): δ = 8,63 (d, J= 2Hz, 1H); 8,03 (d, J = 2Hz, 1H) ppm.

Analyse: $C_6H_2Cl_2F_3M$ (216,0)

berechnet: C 33,36% H 0,93% N 6,48% Cl 32,82% F 26,38%

gefunden: C 33,5% R 1,0% N 6,5% Cl 33,4% F 25,9%.

**Beispiel 4:**

**Einstufige Herstellung von 2,3-Dichlor-5-trifluormethylpyridin**

Der im Beispiel 2 verwendete Ansatz wird in einem Tantal-Autoklaven 2 Stunden lang bei 150°C und anschliessend weitere 2 Stunden lang bei 180° erhitzt. Nach Verdampfen des Lösungsmittels wird der Rückstand mit 50 ml Aether aufgenommen und die ätherische Lösung filtriert. Der Aether wird unter Wasserstrahl-Vakuum abdestilliert und der Rückstand wird einer Wasserdampfdestillation unterworfen. Das resultierende Produkt ist mit dem nach Beispiel 3 erhaltenen Produkt identisch.

## Patentansprüche

1. 2,2-Dichlor-3,3,3-trifluorpropionaldehyd.
2. Verfahren zur Herstellung von 2,2-Dichlor-3 3 3-trifluorpropionaldehyd, dadurch gekennzeichnet, dass man entsprechende Olefine einer Ozonolyse unterzieht und die Reaktionsmasse reduktiv aufarbeitet.

## Claims:

1. 2,2-Dichloro-3 3 3-trifluoropropionaldehyde
2. A process for the preparation of 2,2-dichloro-3,3,3-trifluoropropionaldehyde, which comprises subjecting a corresponding olefin to ozonolysis and working up the reaction mass by reduction.

## Revendications

1. 2,2-dichloro-3,3,3-trifluoropropionaldéhyde.
2. Procédé de préparation de 2,2-dichloro-3,3,3-trifluoropropionaldéhyde, caractérisé en ce qu'on soumet les

oléfines correspondantes à une ozonolyse et en ce qu'on traite la masse réactionnelle de façon réductrice.